# EUROPEAN PATENT SPECIFICATION

(11) **EP 4 525 752 B1**
(45) Date of publication and mention of the grant of the patent: **16.09.2026**
(21) Application number: 23731871.2
(22) Date of filing: 19.05.2023
(51) Int. Cl.: A61B 17/34, A61B 1/00, A61B 1/012, A61B 17/22, A61M 25/01

(54) **SYSTEMS FOR TREATING A STRICTURE ALONG THE BILIARY AND/OR PANCREATIC TRACT**
SYSTEME ZUR BEHANDLUNG EINER VERENGUNG ENTLANG DES GALLEN- UND/ODER PANKREASTRAKTES
SYSTÈMES DE TRAITEMENT D'UNE STÉNOSE LE LONG DES VOIES BILIAIRES ET/OU PANCRÉATIQUES

(30) Priority: 19.05.2022 US 202263343932 P
(43) Date of publication of application: 26.03.2025
(73) Proprietor: Boston Scientific Scimed, Inc., Maple Grove, MN 55311 (US)
(72) Inventor: GESSLER III, Raymond, Roberts, Wisconsin 54023 (US); OLSON, Mark, New Brighton, Minnesota 55112 (US); MATTESON, Jason, Beldenville, Wisconsin 54003 (US); STENGER, Nathan, St. Paul, Minnesota 55102 (US); DAYTON, Peter, Brookline, Massachusetts 02446 (US)
(74) Representative: Vossius & Partner Patentanwälte Rechtsanwälte mbB
(86) International application number: PCT/US2023/022938
(87) International publication number: WO 2023/225321

(56) References cited:
- WO-A1-2022/031763
- WO-A1-98/43530
- US-A- 5 885 288
- US-A- 6 126 633
- US-A1- 2016 361 088
- US-A1- 2020 316 349

## Description

### Technical Field

The present disclosure pertains to medical devices, and methods for manufacturing medical devices. More particularly, the present disclosure pertains to medical devices for treating strictures along the biliary and/or pancreatic tract.

### Background

A wide variety of medical devices have been developed for medical use. Some of these devices include guidewires, catheters, and the like. These devices are manufactured by any one of a variety of different manufacturing methods and may be used according to any one of a variety of methods. Of the known medical devices and methods, each has certain advantages and disadvantages. There is an ongoing need to provide alternative medical devices as well as alternative methods for manufacturing and using medical devices.

WO 2022/031763 A1 discloses systems and methods for treating a stricture, for example along the biliary and/or pancreatic tract.

WO 98/43530 A1 discloses an elongate probe having a longitudinal axis and a distal tip, and including at least one deflection mechanism, which includes an elastic flexible member, having distal and proximal ends and having a predetermined bending stiffness. The flexible member is fixed within the probe generally parallel to the longitudinal axis thereof. The probe further includes a pull wire having a distal end coupled to the distal end of the flexible member, and a proximal end that is tensioned longitudinally to deflect the probe.

### Summary

The invention is defined by a system for treating a stricture according to the independent claim. Preferred embodiments are defined in the dependent claims. This disclosure provides design, material, manufacturing method, and use alternatives for medical devices. A system for treating a stricture is disclosed. The system comprises: a handle having a base having a proximal end region; a length adjustment member coupled to the handle, the length adjustment member having a distal end configured to be attached to an endoscope; wherein the base is movable relative to the length adjustment member; a length actuator coupled to the handle, the length actuator being configured to allow a user to vary the position of the base relative to the length adjustment member; a catheter shaft coupled to the base; a steering mechanism coupled to the handle, the steering mechanism being configured to steer the catheter shaft; and wherein the steering mechanism includes an elongate steering member having a first end region coupled to the handle, a first body region extending along the catheter shaft, a loop region extending about the catheter shaft, a second body region extending along the catheter shaft, and a second end region coupled to the handle.

Alternatively or additionally to any of the embodiments above, the elongate steering member includes a steering wire.

Alternatively or additionally to any of the embodiments above, the elongate steering member includes a multi-filar steering cable.

Alternatively or additionally to any of the embodiments above, the first body region and the second body region extend adjacent to one another along the catheter shaft.

Alternatively or additionally to any of the embodiments above, the first body region, the second body region, or both extend along an outer surface of the catheter shaft.

Alternatively or additionally to any of the embodiments above, the first body region, the second body region, or both extend through a wall of the catheter shaft.

Alternatively or additionally to any of the embodiments above, the first end region and the second end region of the elongate steering member are coupled to a steering connector disposed within the handle.

Alternatively or additionally to any of the embodiments above, the first end region and the second end region of the elongate steering member extend in one or more loop formations at the steering connector.

Alternatively or additionally to any of the embodiments above, the first end region and the second end region of the elongate steering member extend in an arrangement that resembles a figure eight at the steering connector.

Alternatively or additionally to any of the embodiments above, the length actuator includes a depressible button.

Alternatively or additionally to any of the embodiments above, further comprising a needle attachment member releasably coupled to the proximal end region of the handle.

Alternatively or additionally to any of the embodiments above, further comprising a needle releasably coupled to the needle attachment member, the needle being configured to pass through tissue into a position along the biliary and/or pancreatic tract.

Alternatively or additionally to any of the embodiments above, the catheter shaft includes a first channel having the first body region extending therethrough.

Alternatively or additionally to any of the embodiments above, the catheter shaft includes a second channel having the second body region extending therethrough.

Alternatively or additionally to any of the embodiments above, the catheter shaft includes a first coil having a first pitch and a second coil having a second pitch different from the first pitch.

An endoscopic medical device is disclosed. The endoscopic medical device comprises: a length adjustment member; a handle coupled to the length adjustment member and being movable relative to the length adjustment member; a length actuator coupled to the handle, the length actuator being configured to allow a user to vary the position of the handle relative to the length adjustment member; a catheter shaft coupled to the handle; a steering actuator coupled to the handle, the steering actuator being configured to steer the catheter shaft; and an elongate steering member coupled to the steering actuator, the steering member having a first end region coupled to the handle, a first body region extending along the catheter shaft, a loop region extending about the catheter shaft, a second body region extending along the catheter shaft, and a second end region coupled to the handle.

Alternatively or additionally to any of the embodiments above, the first end region and the second end region of the elongate steering member are coupled to the steering actuator.

Alternatively or additionally to any of the embodiments above, the first end region and the second end region of the elongate steering member extend in one or more loop formations adjacent to the steering actuator.

Alternatively or additionally to any of the embodiments above, the first end region and the second end region of the elongate steering member extend in an arrangement that resembles a figure eight adjacent to the steering actuator.

An endoscopic medical device assembly is disclosed. The endoscopic medical device assembly comprises: a handle assembly including a handle and a length adjustment member; a length actuator coupled to the handle assembly, the length actuator being configured to allow a user to vary the position of the handle relative to the length adjustment member; a catheter shaft coupled to the handle; a steering actuator coupled to the handle, the steering actuator being configured to steer the catheter shaft; an elongate steering member coupled to the steering actuator, the steering member having a first end region coupled to the handle, a first body region extending along the catheter shaft, a loop region extending about the catheter shaft, a second body region extending along the catheter shaft, and a second end region coupled to the handle; a needle attachment member releasably coupled to the handle region; and a needle releasably coupled to the needle attachment member, the needle being configured to pass through tissue into a position along the biliary and/or pancreatic tract.

The above summary of some embodiments is not intended to describe each disclosed embodiment or every implementation of the present disclosure. The Figures, and Detailed Description, which follow, more particularly exemplify these embodiments.

### Brief Description of the Drawings

The disclosure may be more completely understood in consideration of the following detailed description in connection with the accompanying drawings, in which:
FIG. 1 is a side view of an example system for accessing the pancreatic and/or biliary tract.
FIG. 2 is a partially cutaway view of a portion of a handle assembly showing an example steering mechanism.
FIG. 3 is a partial cross-sectional view of a portion of an example catheter shaft.
FIG. 4 is a partial cross-sectional view of a portion of an example catheter shaft.
FIG. 5 is a cross-sectional view taken through line 5-5 in FIG. 4.
FIG. 6 illustrates an example needle attachment member and an example needle.
FIG. 7 illustrates an example needle attachment member coupled to an example handle assembly.
FIG. 8 illustrates a portion of an example handle assembly.
FIG. 9 illustrates a portion of an example handle assembly.
FIG. 10 schematically depicts a portion of the digestive system.
FIGS. 11-13 illustrate a method for accessing a stricture according to the present disclosure.
FIG. 14 is a flow chart depicting an example method.

While the disclosure is amenable to various modifications and alternative forms, specifics thereof have been shown by way of example in the drawings and will be described in detail. It should be understood, however, that the intention is not to limit the invention to the particular embodiments described.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (e.g., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

It is noted that references in the specification to "an embodiment", "some embodiments", "other embodiments", etc., indicate that the embodiment described may include one or more particular features, structures, and/or characteristics. However, such recitations do not necessarily mean that all embodiments include the particular features, structures, and/or characteristics. Additionally, when particular features, structures, and/or characteristics are described in connection with one embodiment, it should be understood that such features, structures, and/or characteristics may also be used connection with other embodiments whether or not explicitly described unless clearly stated to the contrary.

The following detailed description should be read with reference to the drawings in which similar elements in different drawings are numbered the same. The drawings, which are not necessarily to scale, depict illustrative embodiments and are not intended to limit the scope of the invention.

In endoscopy, a frequent medical condition arises when a patient presents with abdominal pain with or without associated jaundice. The etiology is usually some type of obstruction in the biliary tree which prevents bile from flowing naturally from the proximal tree into the duodenum. The blockage may be the result of biliary stones caught in the lumen of the ducts or a tumor which is either in the wall of the duct or impinging upon the wall from adjacent tissue. When such a stricture occurs the duct proximal to the stricture dilates and the duct distal to the stricture receives a reduced flow of bile. In order to relieve the patient's symptoms, gastroenterologists seek to find a method for resuming the flow of bile from the proximal dilated duct into the duodenum. Some interventions contemplated for reliving symptoms may include placing a stent across the stricture to drain the proximal duct, removing a stone, and/or the like.

The most common method of placing a stent across the stricture is to perform an endoscopic retrograde cholangio-pancreatography (ERCP) where a side-viewing endoscope is placed in the duodenum at the location of the biliary papilla and a guidewire is placed through the papilla and up the biliary duct, across the stricture, in a retrograde fashion. Such procedures may be challenging. For example, depending on the location, geometry, and mechanics of the stricture, deep cannulation of the proximal duct may be difficult if not be possible. Furthermore, when the physician attempts to access the biliary duct, they may inadvertently cannulate the pancreatic duct. Inadvertent cannulation of the pancreatic duct could lead to complications such as pancreatitis. Disclosed herein are devices and methods that address these and other issues, for example by utilizing antegrade (e.g., non-papillary) stricture crossing.

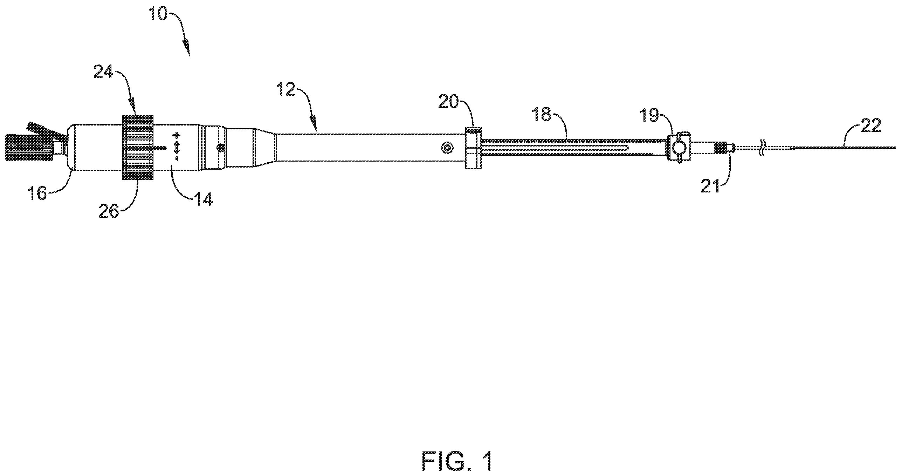

FIG. 1 illustrates an example system 10 for treating a stricture along the biliary and/or pancreatic tract. In at least some instances, the system 10 is configured to be used with an endoscope (e.g., and/or with an endoscopic ultrasound device) for antegrade stricture crossing along the biliary and/or pancreatic tract. As will be described in more detail herein, the system 10 may be advanced through at least a portion of the digestive tract of a patient, used to pierce/puncture through a wall of the digestive tract and into a lumen/duct along the biliary and/or pancreatic tract, steered to a desired orientation, and then be used to allow a suitable device to pass therethrough for an intervention.

The system 10 may include a handle or handle assembly 12. The handle assembly 12 may include a base 14 having a proximal end region 16. A length adjustment member 18 may be coupled to the handle assembly 12. For example, the length adjustment member 18 may be coupled to the base 14 and be movable relative thereto. In practice, a user may attach the length adjustment member 18 to an endoscope (e.g., as disclosed herein) and the handle assembly 12 (and/or base 14) can be moved in order to manipulate a device (e.g., a catheter shaft, needle, or both) relative to the length adjustment member 18 (and/or the endoscope). In general, the length adjustment member 18 may be configured to adjust the length or "throw" of a device (e.g., a catheter shaft, needle, or both) relative to an endoscope (e.g., how far the device may be advanced from an endoscope toward a target location). A length adjustment actuator 20 may be coupled to the handle assembly 12. In general, the length adjustment actuator 20 may be configured to allow a user to vary the position of the handle assembly 12 (and/or the base 14) relative to the length adjustment member 18. In at least some instances, the length adjustment actuator 20 may take the form of a depressible button. In use, the length adjustment actuator 20 can be pressed to allow the base 14 to be shifted relative to the length adjustment member 18. When the length adjustment member 18 is secured to an endoscope and when the base 14 is secured to a device (e.g., a catheter shaft, needle, or both), actuating the length adjustment actuator 20 allows the device to be shifted (e.g., translated) relative to the endoscope. Releasing the length adjustment actuator 20 may secure the length adjustment member 18 (and/or the endoscope) relative to the base 14 (and/or the device). Some additional details pertaining to the length adjustment actuator 20 are disclosed herein.

The handle assembly 12 may include a rotation member 19. In general, the rotation member 19 may be used to manipulate the handle assembly 12 (and/or the base 14) relative to the length adjustment member 18. This may include rotation of the handle assembly 12 (and/or the base 14) relative to the length adjustment member 18. The handle assembly 12 may include an endoscope connector 21, which may be disposed adjacent to the rotation member 19. The endoscope connector 21 may allow the handle assembly 12 to be attached to an endoscope.

A catheter shaft 22 may be coupled to the handle assembly 12 (and/or the base 14) and be movable therewith. In general, the catheter shaft 22 may be configured to extend through a channel in an endoscope, exit out from the endoscope and through at least a portion of the digestive tract of a patient, extend through a wall of the digestive tract and into a lumen/duct along the biliary and/or pancreatic tract (e.g., which may include the use of a needle or other "sharp" device), and be steered (e.g., curved, bent and/or articulated) to a desired orientation, and then be used to allow a suitable device to pass therethrough as part of an intervention. Some additional details pertaining to the catheter shaft 22 are disclosed herein.

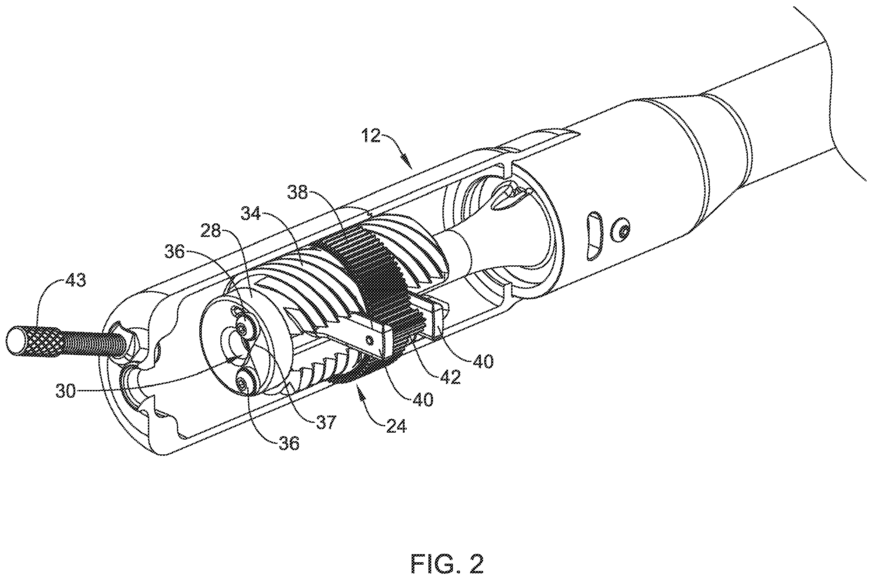

The handle assembly 12 may also include steering mechanism 24. In general, the steering mechanism 24 may be configured to steer the catheter shaft 22. The steering mechanism 24 may include a steering actuator 26, a steering connector 28 (e.g., generally disposed within the handle assembly 12, not shown in FIG. 1 but can be seen in FIG. 2), and a steering member 30. The steering actuator 26 may include a knob or button disposed along the handle assembly 12 (not shown in FIG. 1 but can be seen in FIG. 2). When actuated, the steering actuator 26 can manipulate the steering member 30 so that the catheter shaft 22 can take a bend or curve. The steering member 30 may take the form of one or more wires or cables (e.g., a multi-filar cable or cables) that extend along the catheter shaft 22 and couple to the steering actuator 26. In some instances, the steering member 30 takes the form of a singular wire or cable (e.g., a single multi-filar cable) that extends distally along the catheter shaft 22 from the handle assembly 12, loops about a distal end region of the catheter shaft 22, extends proximally along catheter shaft 22 toward the handle assembly 12. The two ends of the cable steering member 30 may then be attached to the steering connector 28 as disclosed herein.

In some instances, the cable may comprise a multi-filar cable formed from or including a material such as tungsten (and/or other materials disclosed herein). In at least some instances, a multi-filar cable may be desirable for a number of reasons. For example, a multi-filar cable may have increased resistance to elongation (e.g., when compared to a monofilament wire), increased flexibility, increased durability, combinations thereof, and/or the like.

FIG. 2 illustrates the steering connector 28. Here it can be seen that the steering connector 28 may take the form of a spool or spool member with an external thread 34. The ends of the steering member 30 may be coupled to the steering connector 28, for example at one or more bolts or connectors 36. For example, when the steering member 30 takes the form of a singular cable that extends down the catheter shaft 22, loops about the catheter shaft 22, and extends back to the steering connector 28, the ends of the cable may be secured to the steering connector 28 at the connectors 36. In at least some instances, the steering member 30 may be looped about or otherwise include one or more looped regions at or adjacent the connectors 36. For example, the steering member 30 may be arranged in a figure eight configuration about the connectors 36. This arrangement may be desirable for a number of reasons. For example, the looped or figure eight arrangement may help to create a strain relief in the steering member 30 while reducing/minimizing the likelihood that a weak point or pinch point will be formed.

The external thread 34 of the steering connector 28 may be threadably engaged with a threaded gear 38. A pair of arms 40 may extend through the steering connector 28 to another gear 42, which is engaged with the threaded gear 38. The arms 40 may extend through an opening the base 14 of the handle assembly 12 so that the gear 42 can engage the steering actuator 26. Thus, actuation of the steering actuator 26 may rotate the gear 42, which rotates the threaded gear 38. Due to the threaded relationship between the threaded gear 38 and the steering connector 28, rotation of the threaded gear 38 caused the steering connector 28 to translate within the handle assembly 12, thereby lengthening or shorting the steering member 30 and steering the catheter shaft 22 (e.g., causing the catheter shaft 22 to take a curve of bend).

In some instances, a steering member limiter 43 may be disposed adjacent to the steering connector 28. The steering member limiter 43 may take the form of a threaded member such as a bolt or screw that can be function as a physical barrier that can engage the steering connector 28 (e.g., when the steering connector 28 translated into engagement with the steering member limiter 43) and prevent further translation of the steering connector 28. This may help to reduce the likelihood that the steering member 30 is over-actuated (e.g., which may include stretching, breaking, and/or other damage), the catheter shaft 22 is over-actuated, combinations thereof, and/or the like. In some instances, the steering member limiter 43 can be adjusted. For example, in instances where the steering member limiter takes the form of a bolt or screw, adjustment may include threading the steering member limiter 43 to a greater or less extent.

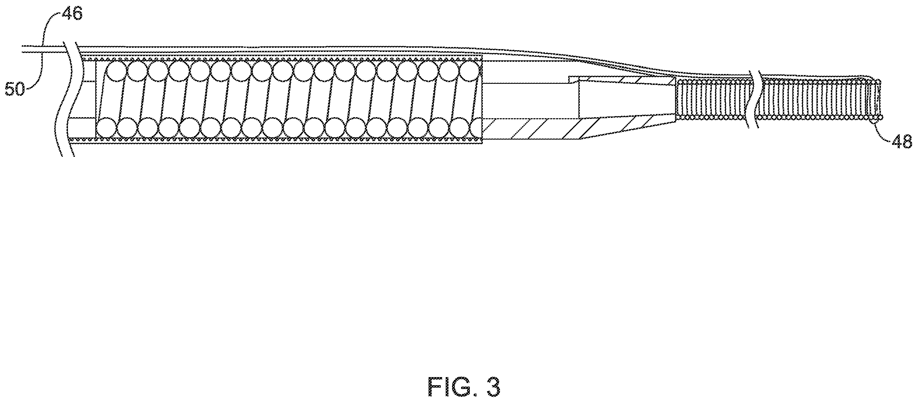

FIG. 3 illustrates the steering member 30 relative to the catheter shaft 22. The steering member may include a first end region coupled to the handle assembly 12 (e.g., at or adjacent to the steering connector 28 as disclosed herein), a first body region 46 extending along the catheter shaft 22, a loop region 48 extending about the catheter shaft 22, a second body region 50 extending along the catheter shaft 22, and a second end region coupled to the handle assembly 12 (e.g., at or adjacent to the steering connector 28 as disclosed herein). The body regions 46, 50 may extend next to one another in a side-by-side arrangement. Portions of the steering member 30 (e.g., the body regions 46, 50) may extend along an outer surface of the catheter shaft 22. In some of these and in other instances, portions of the steering member 30 (e.g., the body regions 46, 50) may extend through the wall of the catheter shaft 22.

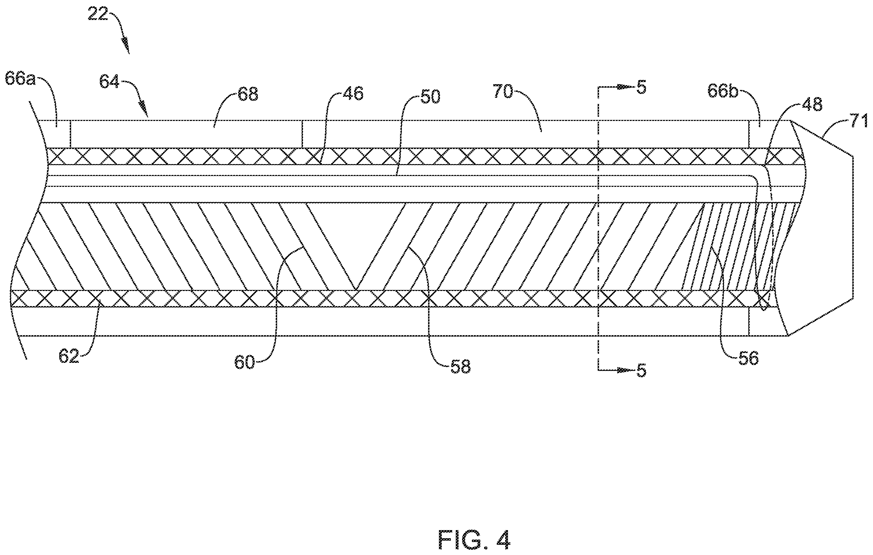

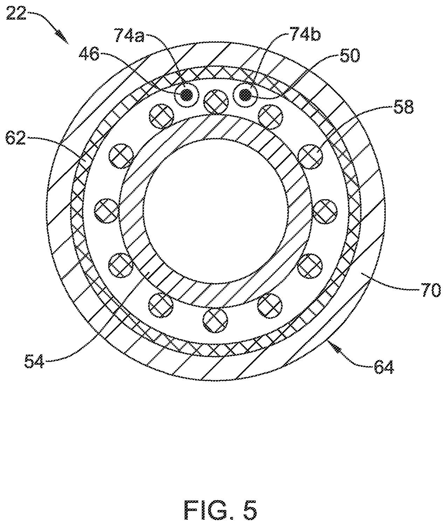

FIGS. 4-5 schematically depict the catheter shaft 22. Here it can be seen that the catheter shaft 22 may include an inner layer or liner 54. A number of different coiled sections may be disposed along the inner liner 54 (not shown in FIG. 4, can be seen in FIG. 5) including a first coil 56, a second coil 58 and a third coil 60. In some instances, the coils 56, 58, 60 may be separate, discrete coils. In other instances, one or more of the coils 56, 58, 60 may be continuous with one another and/or overlap with one another. It can be seen that the winding direction and pitch may vary. For example, the first coil 56 may have a distally oriented or angled winding direction with a relatively close or tight pitch. The second coil 58 may wind in the same direction but have a different, relatively loose or open pitch. The third coil 60 may wind in a different, proximally oriented or angled direction. A support member or braid 62 may be disposed over the coils 56, 58, 60.

A sleeve or jacket 64 may be disposed over the braid 62. The jacket 64 may include end regions 66a, 66b. The end regions 66a, 66b may be relatively short regions that are configured to engage the ends of the braid 62 and that may help to keep the braid 62 intact. In some instances, the end regions 66a, 66b may include nylon-12. In some of these and in other instances, the end regions 66a, 66b may include other materials such as those disclosed herein. The jacket 64 may also include one or more intermediate sections including a first intermediate region 68 and a second intermediate region 70. In some instances, the intermediate regions 68, 70 may include a relatively flexible polymer material such as a polyether block amide. In some instances, the intermediate regions 68, 70 may have different durometers. For example, the intermediate region 68 may include a 40D polyether block amide and the intermediate region 70 may include a 55D polyether block amide. Other materials and durometers are contemplated. A tip 71 may be disposed at or formed at a distal end of the catheter shaft 22.

As shown in FIG. 5, one or more tubes or channels 74a, 74b may be formed in the catheter shaft 22. The channels 74a, 74b may be configured to have the body regions 46, 50 of the steering member 30 extend therethrough. In general, the channels 74a, 74b may be disposed underneath the braid 72. However, other locations are contemplated.

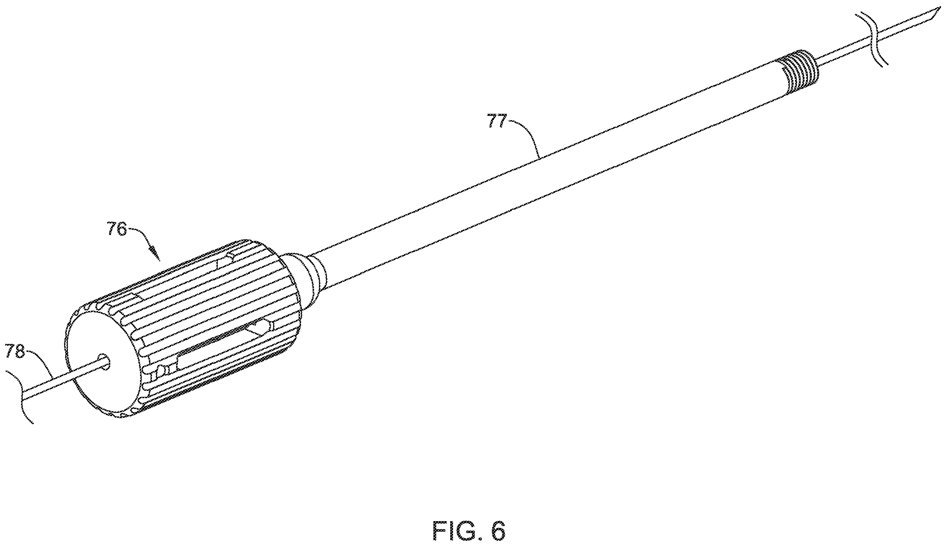

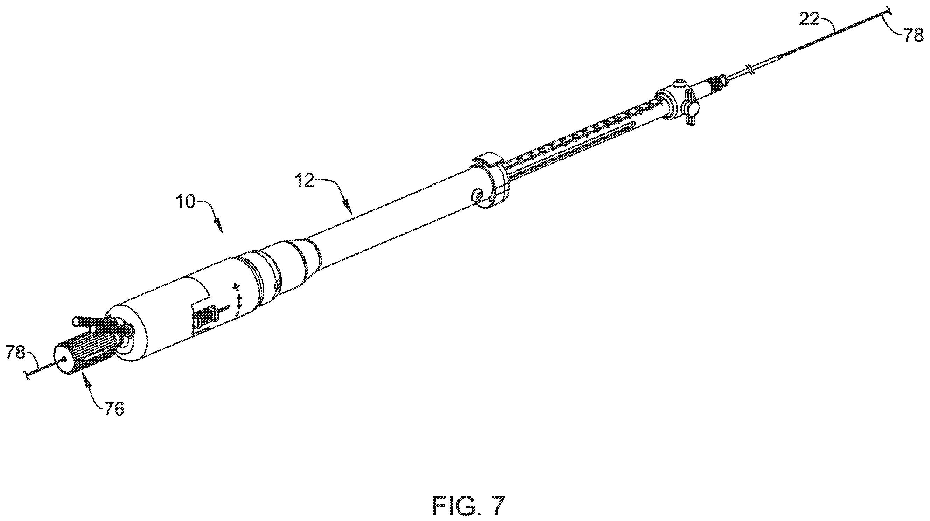

A needle attachment member 76 that is configured to be releasably coupled to the proximal end region of the handle assembly 12 is shown in FIG. 6. A needle or sharp 78 may be releasably secured to the needle attachment member 76. For example, the needle attachment member 76 may include a collet, set screw(s), and/or other mechanism to secure the needle 78 thereto. A threaded stem 77 may extend from the needle attachment member 76. The stem 77 may allow the needle attachment member 76 to be secured to the proximal end region 16 of the handle assembly 12 (e.g., as shown in FIG. 7).

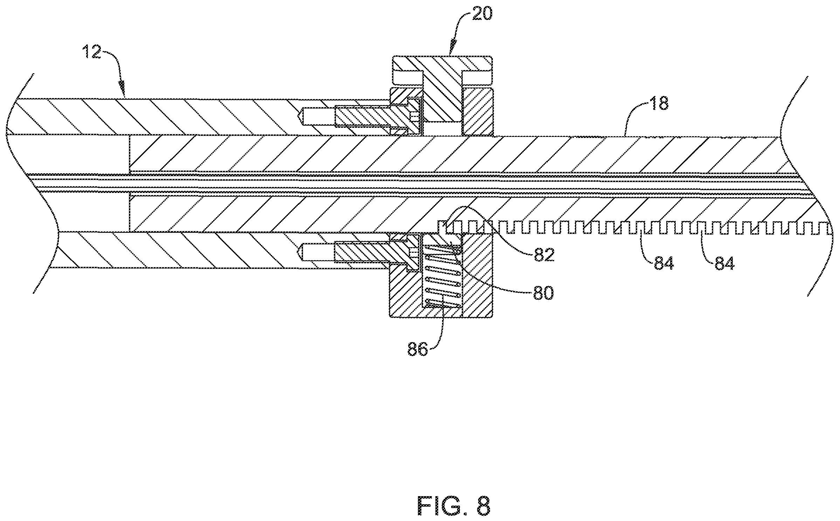

The length adjustment actuator 20 is shown in FIG. 8. Here it can be seen the length adjustment actuator 20 includes an engagement region 80 with a projection 82. The projection 82 is configured to engage one of a number of grooves 84 formed in the length adjustment member 18. A biasing member or spring 86 may urge the engagement region 80 so that the projection 82 engages one of the grooves 84. In general, the spring 86 may bias the projection 82 of the engagement region 80 into engagement with the grooves 84. Depressing the length adjustment actuator 20 overcome the bias and frees the engagement region 80 from engagement with the grooves 84.

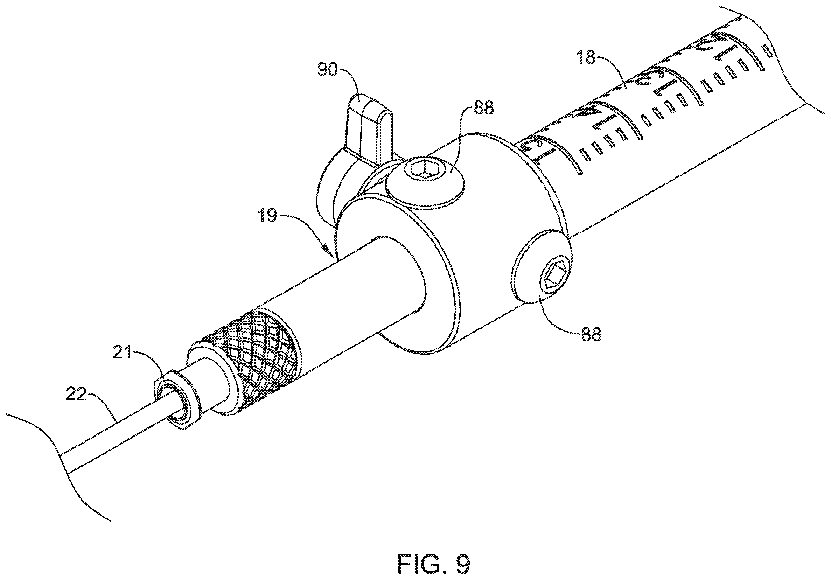

The rotation member 19 is shown in FIG. 9. Here it can be seen that the rotation member 19 includes one or more torque limiters 88 and a locking member 90. The torque limiters 88 may be used to prevent the rotation member 19 from rotating continuously relative to the handle assembly 12. For example, the torque limiters 88 may set a rotation range that sets the amount of rotation to an angle less than 360 degrees. Once the rotational aligned of the rotation member 19 is oriented in the desired manner, the locking member 90 may be actuated to set/lock the rotational alignment.

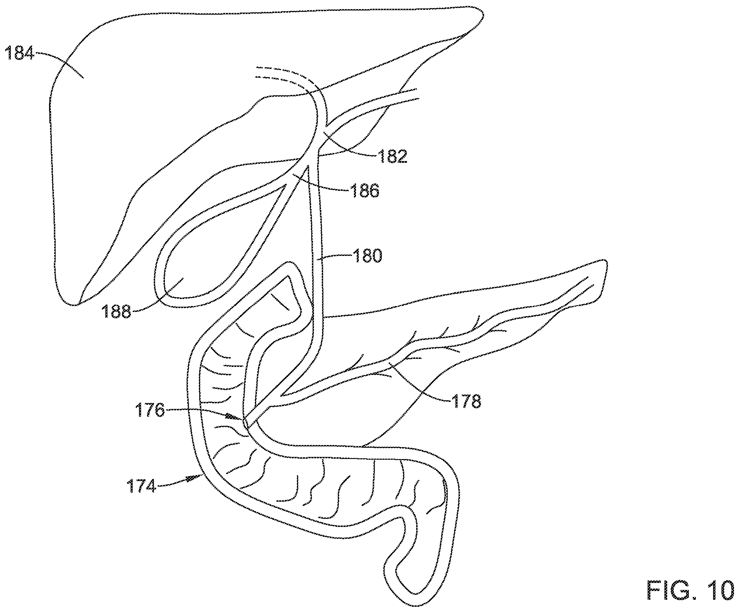

FIG. 10 illustrates an overview of the biliary system or tree. A portion of the duodenum 174 is shown. The papilla of Vater 176 (e.g., also known as the ampulla of Vater or simply the papilla) is located at the illustrated portion of the duodenum 174. The papilla 176 generally forms the opening where the pancreatic duct 178 and the common bile duct 180 can empty into the duodenum 174. The hepatic ducts, denoted by the reference numeral 182, are connected to the liver 184 and empty into the bile duct 180. Similarly, the cystic duct 186, being connected to the gall bladder 188, also empties into the bile duct 180. In general, an endoscopic or biliary procedure may include advancing a medical device to a suitable location along the biliary tree and then performing the appropriate intervention.

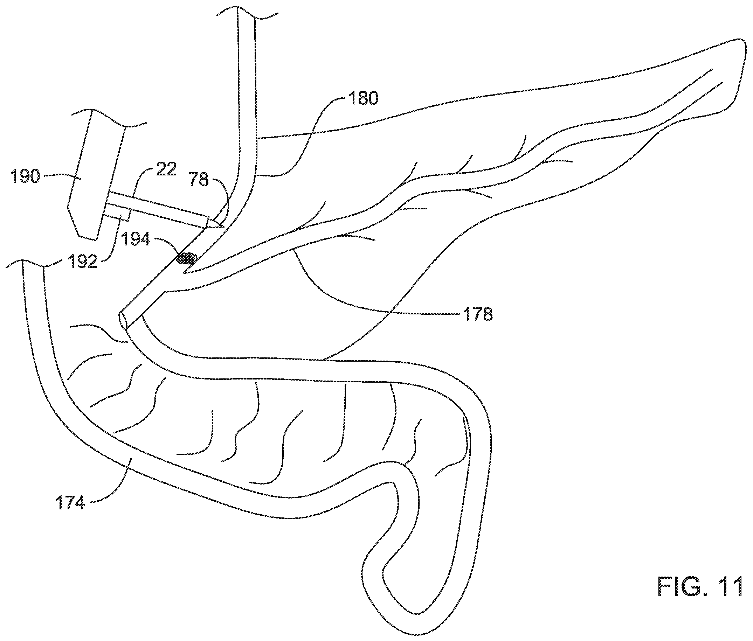

System 10 may be used to access a stricture along the pancreatic duct 178 and/or the common bile duct 180. For example, FIG. 11 illustrates an example endoscope 190 disposed in the duodenum 174. The disclosure is not intended to be limited to disposing the endoscope 190 in the duodenum 174 as other locations are contemplated including other portions of the digestive system including the stomach. The system 10 may be secured to the endoscope 190 with the catheter shaft 22 extending through a channel (e.g., a working channel) of the endoscope 190, and the handle assembly 12 may be manipulated to position the catheter shaft 22. The catheter shaft 22 (e.g., having the needle/sharp 78 extending therethrough) may be advanced out from the endoscope 190. This may include the use of an elevator 192 of the endoscope 190 to direct the catheter shaft 22 toward the wall of the duodenum 174. The catheter shaft 22 having the needle/sharp 78 protruding therefrom may then pierce through the wall of the duodenum 174 and advance through tissue toward the target location, for example, a luminal position such as within common bile duct 180.

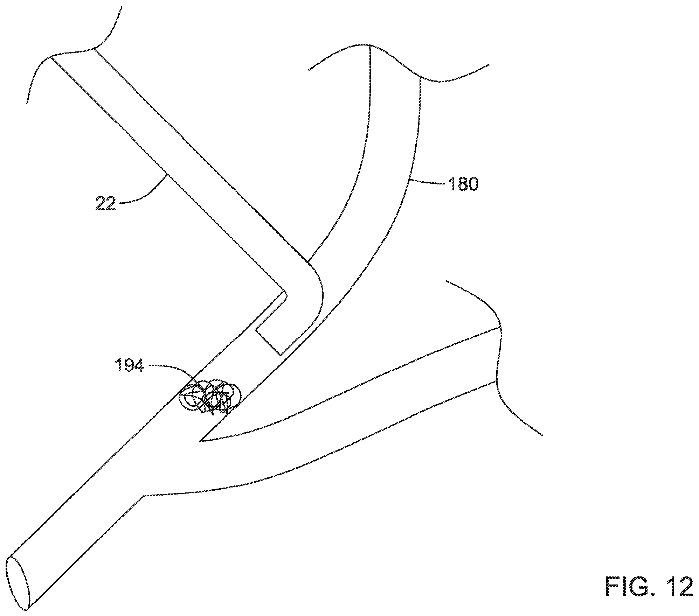

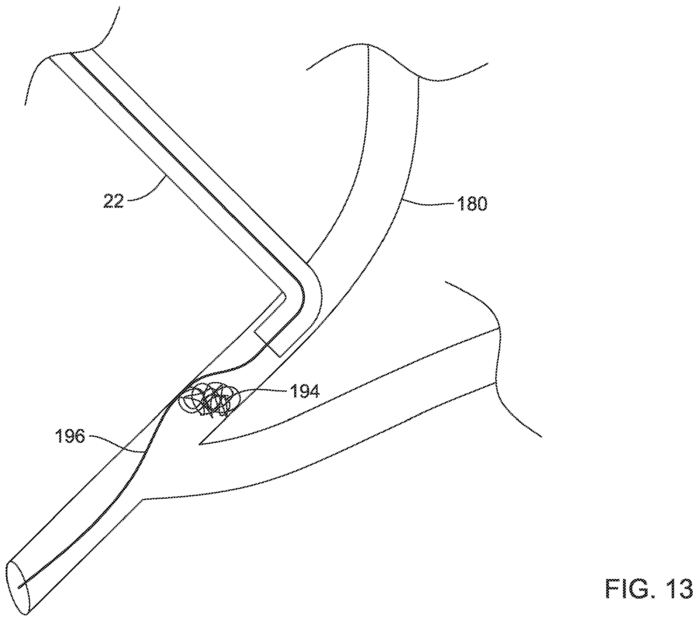

Once the catheter shaft 22 is suitably positioned, the catheter shaft 22 can be steered to the desired orientation using the steering mechanism 24 as schematically depicted in FIG. 12. In at least some instances, it may be desirable to remove the needle/sharp 78 from the system 10 prior to steering. Once the catheter shaft 22 is suitably oriented, a device such as a guidewire 196 may be advanced through the catheter shaft 122 and past the stricture 194 as depicted in FIG. 13.

The materials that can be used for the various components of the system 10 may include those commonly associated with medical devices. For simplicity purposes, the following discussion makes reference to the catheter shaft 22. However, this is not intended to limit the devices and methods described herein, as the discussion may be applied to other similar tubular members and/or components of tubular members or devices disclosed herein.

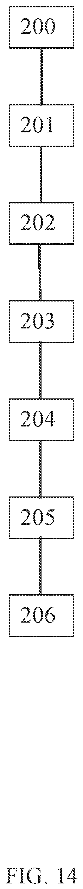

FIG. 14 is a flowchart depicting an example method. The method may include disposing an endoscope 190 in the digestive system (e.g., the duodenum 174) as represented by box 200. A system like system 10 may be secured to the endoscope 190 with the catheter shaft 22 extending through a channel (e.g., a working channel) of the endoscope 190 as represented by box 201. The handle assembly 12 may be manipulated to position the catheter shaft 22 as represented by box 202. The catheter shaft 22 (e.g., having the needle/sharp 78 extending therethrough) may be advanced out from the endoscope 190 as represented by box 203. This may include the use of an elevator 192 of the endoscope 190 to direct the catheter shaft 22 toward the wall of the duodenum 174. The catheter shaft 22 having the needle/sharp 78 protruding therefrom may then pierce through the wall of the duodenum 174 and advance through tissue toward the target location, for example, a luminal position such as within common bile duct 180 as represented by box 204. Once the catheter shaft 22 is suitably positioned, the catheter shaft 22 can be steered to the desired orientation using the steering mechanism 24 as represented by box 205. In at least some instances, it may be desirable to remove the needle/sharp 78 from the system 10 prior to steering. Once the catheter shaft 22 is suitably oriented, a device such as a guidewire 196 may be advanced through the catheter shaft 122 and past the stricture 194 as represented by box 206.

The catheter shaft 22 may be made from or otherwise includes a metal, metal alloy, polymer (some examples of which are disclosed below), a metal-polymer composite, ceramics, combinations thereof, and the like, or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), high-density polyethylene, low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. In some embodiments the sheath can be blended with a liquid crystal polymer (LCP). For example, the mixture can contain up to about 6 percent LCP.

Some examples of suitable metals and metal alloys include stainless steel, such as 304V, 304L, and 316LV stainless steel; mild steel; nickel-titanium alloy such as linear-elastic and/or super-elastic nitinol; other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R30035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickel-cobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R30003 such as ELGILOY^{®}, PHYNOX^{®}, and the like); platinum enriched stainless steel; titanium; combinations thereof; and the like; or any other suitable material.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention is defined by the following claims.

## Claims

1. A system (10) for treating a stricture (194), the system (10) comprising:
a handle (12) having a base (14) having a proximal end region (16);
a length adjustment member (18) coupled to the handle (12), the length adjustment member (18) having a distal end configured to be attached to an endoscope (190);
wherein the base (14) is movable relative to the length adjustment member (18);
a length actuator coupled to the handle (12), the length actuator being configured to allow a user to vary the position of the base (14) relative to the length adjustment member (18);
a catheter shaft (22, 122) coupled to the base (14);
a steering mechanism (24) coupled to the handle (12), the steering mechanism (24) being configured to steer the catheter shaft (22, 122); **characterised in that** the steering mechanism (24) includes an elongate steering member having a first end region coupled to the handle (12), a first body region (46) extending along the catheter shaft (22, 122), a loop region (48) extending about the catheter shaft (22, 122), a second body region (50) extending along the catheter shaft (22, 122), and a second end region coupled to the handle (12).

2. The system (10) of claim 1, wherein the elongate steering member includes a steering wire.

3. The system (10) of any one of claims 1-2, wherein the elongate steering member includes a multi-filar steering cable.

4. The system (10) of any one of claims 1-3, wherein the first body region (46) and the second body region (50) extend adjacent to one another along the catheter shaft (22, 122).

5. The system (10) of any one of claims 1-4, wherein the first body region (46), the second body region (50), or both extend along an outer surface of the catheter shaft (22, 122).

6. The system (10) of any one of claims 1-5, wherein the first body region (46), the second body region (50), or both extend through a wall of the catheter shaft (22, 122).

7. The system (10) of any one of claims 1-6, wherein the first end region and the second end region of the elongate steering member are coupled to a steering connector (28) disposed within the handle (12).

8. The system (10) of claim 7, wherein the first end region and the second end region of the elongate steering member extend in one or more loop formations at the steering connector (28).

9. The system (10) of any one of claims 7-8, wherein the first end region and the second end region of the elongate steering member extend in an arrangement that resembles a figure eight at the steering connector (28).

10. The system (10) of any one of claims 1-9, wherein the length actuator includes a depressible button.

11. The system (10) of any one of claims 1-10, further comprising a needle attachment member (76) releasably coupled to the proximal end region (16) of the handle (12).

12. The system (10) of claim 11, further comprising a needle (78) releasably coupled to the needle attachment member (76), the needle (78) being configured to pass through tissue into a position along the biliary and/or pancreatic tract.

13. The system (10) of any one of claims 1-12, wherein the catheter shaft (22, 122) includes a first channel having the first body region (46) extending therethrough.

14. The system (10) of claim 13, wherein the catheter shaft (22, 122) includes a second channel having the second body region (50) extending therethrough.

15. The system (10) of any one of claims 1-14, wherein the catheter shaft (22, 122) includes a first coil (56) having a first pitch and a second coil (58) having a second pitch different from the first pitch.

## Patentansprüche

1. System (10) zum Behandeln einer Striktur (194), wobei das System (10) aufweist:
einen Griff (12) mit einer Basis (14), die einen proximalen Endbereich (16) aufweist;
ein mit dem Griff (12) verbundenes Längenverstellelement (18), wobei das Längenverstellelement (18) ein distales Ende aufweist, das dafür konfiguriert ist, an einem Endoskop (190) verbunden zu werden,
wobei die Basis (14) relativ zum Längenverstellelement (18) beweglich ist;
einen mit dem Griff (12) verbundenen Längenaktuator, wobei der Längenaktuator dafür konfiguriert ist, einem Benutzer zu ermöglichen, die Position der Basis (14) relativ zum Längenverstellelement (18) zu verändern;
einen mit der Basis (14) verbundenen Katheterschaft (22, 122);
einen mit dem Griff (12) verbundenen Lenkmechanismus (24), wobei der Lenkmechanismus (24) dafür konfiguriert ist, den Katheterschaft (22, 122) zu lenken,
**dadurch gekennzeichnet, dass**
der Lenkmechanismus (24) aufweist: ein längliches Lenkelement mit einem ersten Endbereich, der mit dem Griff (12) verbunden ist, einem ersten Körperbereich (46), der sich entlang des Katheterschafts (22, 122) erstreckt, einem Schleifenbereich (48), der sich um den Katheterschaft (22, 122) herum erstreckt, einem zweiten Körperbereich (50), der sich entlang des Katheterschafts (22, 122) erstreckt, und einem zweiten Endbereich, der mit dem Griff (12) verbunden ist.

2. System (10) nach Anspruch 1, wobei das längliche Lenkelement einen Lenkdraht aufweist.

3. System (10) nach Anspruch 1 oder 2, wobei das längliche Lenkelement ein mehrsträngiges Lenkseil aufweist.

4. System (10) nach einem der Ansprüche 1 bis 3, wobei sich der erste Körperbereich (46) und der zweite Körperbereich (50) benachbart zueinander entlang des Katheterschafts (22, 122) erstrecken.

5. System (10) nach einem der Ansprüche 1 bis 4, wobei sich der erste Körperbereich (46) und/oder der zweite Körperbereich (50) entlang einer Außenfläche des Katheterschafts (22, 122) erstrecken.

6. System (10) nach einem der Ansprüche 1 bis 5, wobei sich der erste Körperbereich (46) und/oder der zweite Körperbereich (50) durch eine Wand des Katheterschafts (22, 122) erstrecken.

7. System (10) nach einem der Ansprüche 1 bis 6, wobei der erste Endbereich und der zweite Endbereich des länglichen Lenkelements mit einem innerhalb des Griffs (12) angeordneten Lenkverbinder (28) verbunden sind.

8. System (10) nach Anspruch 7, wobei sich der erste Endbereich und der zweite Endbereich des länglichen Lenkelements in einer oder mehreren Schleifenformationen am Lenkverbinder (28) erstrecken.

9. System (10) nach Anspruch 7 oder 8, wobei sich der erste Endbereich und der zweite Endbereich des länglichen Lenkelements in einer Anordnung am Lenkverbinder (28) erstrecken, die achtförmig ist.

10. System (10) nach einem der Ansprüche 1 bis 9, wobei der Längenaktuator einen drückbaren Knopf aufweist.

11. System (10) nach einem der Ansprüche 1 bis 10, ferner aufweisend ein Nadelbefestigungselement (76), das lösbar mit dem proximalen Endbereich (16) des Griffs (12) verbunden ist.

12. System (10) nach Anspruch 11, ferner aufweisend eine Nadel (78), die lösbar mit dem Nadelbefestigungselement (76) verbunden ist, wobei die Nadel (78) derart konfiguriert ist, dass sie durch Gewebe hindurch in eine Position entlang des Gallen- und/oder Pankreasgangs führt.

13. System (10) nach einem der Ansprüche 1 bis 12, wobei der Katheterschaft (22, 122) einen ersten Kanal aufweist, durch den sich der erste Körperbereich (46) erstreckt.

14. System (10) nach Anspruch 13, wobei der Katheterschaft (22, 122) einen zweiten Kanal aufweist, durch den sich der zweite Körperbereich (50) erstreckt.

15. System (10) nach einem der Ansprüche 1 bis 14, wobei der Katheterschaft (22, 122) eine erste Wendel (56) mit einer ersten Steigung und eine zweite Wendel (58) mit einer von der ersten Steigung verschiedenen zweiten Steigung aufweist.

## Revendications

1. Système (10) pour traiter une sténose (194), le système (10) comprenant :
une poignée (12) ayant une base (14) ayant une région d'extrémité proximale (16) ;
un élément de réglage de longueur (18) couplé à la poignée (12), l'élément de réglage de longueur (18) ayant une extrémité distale configurée pour être fixée à un endoscope (190) ;
dans lequel la base (14) est mobile par rapport à l'élément de réglage de longueur (18) ;
un actionneur de longueur couplé à la poignée (12), l'actionneur de longueur étant configuré pour permettre à un utilisateur de modifier la position de la base (14) par rapport à l'élément de réglage de longueur (18) ;
une tige de cathéter (22, 122) couplée à la base (14) ;
un mécanisme de guidage (24) couplé à la poignée (12), le mécanisme de guidage (24) étant configuré pour guider la tige de cathéter (22, 122) ;
**caractérisé en ce que** :
le mécanisme de guidage (24) comprend un élément de guidage allongé ayant une première région d'extrémité couplée à la poignée (12), une première région de corps (46) s'étendant le long de la tige de cathéter (22, 122), une région de boucle (48) s'étendant autour de la tige de cathéter (22, 122), une deuxième région de corps (50) s'étendant le long de la tige de cathéter (22, 122), et une deuxième région d'extrémité couplée à la poignée (12).

2. Système (10) selon la revendication 1, dans lequel l'élément de guidage allongé comprend un fil de guidage.

3. Système (10) selon l'une quelconque des revendications 1 à 2, dans lequel l'élément de guidage allongé comprend un câble de guidage multifilaire.

4. Système (10) selon l'une quelconque des revendications 1 à 3, dans lequel la première région de corps (46) et la deuxième région de corps (50) s'étendent de manière adjacente l'une par rapport à l'autre le long de la tige de cathéter (22, 122).

5. Système (10) selon l'une quelconque des revendications 1 à 4, dans lequel la première région de corps (46), la deuxième région de corps (50) ou les deux s'étendent le long d'une surface externe de la tige de cathéter (22, 122).

6. Système (10) selon l'une quelconque des revendications 1 à 5, dans lequel la première région de corps (46), la deuxième région de corps (50) ou les deux s'étendent à travers une paroi de la tige de cathéter (22, 122).

7. Système (10) selon l'une quelconque des revendications 1 à 6, dans lequel la première région d'extrémité et la deuxième région d'extrémité de l'élément de guidage allongé sont couplées à un connecteur de guidage (28) disposé à l'intérieur de la poignée (12).

8. Système (10) selon la revendication 7, dans lequel la première région d'extrémité et la deuxième région d'extrémité de l'élément de guidage allongé s'étendent dans une ou plusieurs formations de boucle au niveau du connecteur de guidage (28).

9. Système (10) selon l'une quelconque des revendications 7 à 8, dans lequel la première région d'extrémité et la deuxième région d'extrémité de l'élément de guidage allongé s'étendent dans un agencement selon une configuration en forme de huit au niveau du connecteur de guidage (28).

10. Système (10) selon l'une quelconque des revendications 1 à 9, dans lequel l'actionneur de longueur comprend un bouton pouvant être enfoncé.

11. Système (10) selon l'une quelconque des revendications 1 à 10, comprenant en outre un élément de fixation d'aiguille (76) couplé, de manière amovible, à la région d'extrémité proximale (16) de la poignée (12).

12. Système (10) selon la revendication 11, comprenant en outre une aiguille (78) couplée, de manière amovible, à l'élément de fixation d'aiguille (76), l'aiguille (78) étant configurée pour traverser le tissu dans une position le long des voies biliaires et/ou pancréatiques.

13. Système (10) selon l'une quelconque des revendications 1 à 12, dans lequel la tige de cathéter (22, 122) comprend un premier canal ayant la première région de corps (46) s'étendant à travers ce dernier.

14. Système (10) selon la revendication 13, dans lequel la tige de cathéter (22, 122) comprend un deuxième canal ayant la deuxième région de corps (50) s'étendant à travers ce dernier.

15. Système (10) selon l'une quelconque des revendications 1 à 14, dans lequel la tige de cathéter (22, 122) comprend une première spire (56) présentant un premier pas et une deuxième spire (58) présentant un deuxième pas différent du premier pas.
